# EUROPEAN PATENT SPECIFICATION

(11) **EP 2 004 827 B1**
(45) Date of publication and mention of the grant of the patent: **10.03.2010**
(21) Application number: 07726886.0
(22) Date of filing: 14.03.2007
(51) Int. Cl.: C12N 15/80, C12N 15/90

(54) **IMPROVED METHOD FOR HOMOLOGOUS RECOMBINATION IN FUNGAL CELLS**
VERBESSERTES VERFAHREN ZUR HOMOLOGEN REKOMBINATION IN PILZZELLEN
PROCÉDÉ AMÉLIORÉ DE RECOMBINAISON HOMOLOGUE DANS DES CELLULES FONGIQUES

(30) Priority: 08.04.2006 EP 06075904
(43) Date of publication of application: 24.12.2008
(73) Proprietor: DSM IP Assets B.V., 6411 TE Heerlen (NL)
(72) Inventor: BERG, VAN DEN, Marco Alexander, NL-2685 EH Poeldijk (NL); OFFRINGA, Remko, NL-3453 ML De Meern (NL); BOER, DE, Paulo, 8245 ED Lelystad (NL)
(74) Representative: Matulewicz, Emil Rudolf Antonius
(86) International application number: PCT/EP2007/052393
(87) International publication number: WO 2007/115887

(56) References cited:
- WO-A-99/53017
- US-A1- 2005 181 509

### SEQUENCE LISTING

<110> DSM IP Assets B.V.
<120> IMPROVED METHOD FOR HOMOLOGOUS RECOMBINATION IN FUNGAL CELLS
<130> 25554WO
<160> 23
<170> PatentIn version 3.1
<210> 1
   <211> 31
   <212> DNA
   <213> Artificial sequence
<220>
   <223> Description of artificial sequence: synthetic primer
<400> 1
   aatattcgaa ttcgagctct gtacagtgac c 31
<210> 2
   <211> 47
   <212> DNA
   <213> Artificial sequence
<220>
   <223> Description of artificial sequence: synthetic primer
<400> 2
   attaggatcc gcggccgcta ttccatggca agctgcgatg aagtggg 47
<210> 3
   <211> 34
   <212> DNA
   <213> Artificial sequence
<220>
   <223> Description of artificial sequence: synthetic primer
<400> 34
   ccttctcgag ccatggatcc tgatgatgtt gttg 34
<210> 4
   <211> 43
   <212> DNA
   <213> Artificial sequence
<220>
   <223> Description of artificial sequence: synthetic primer
<400> 4
   ccaagtcgac aagcttcatc gcctgacacg atttcctgca cag 43
<210> 5
   <211>
   <212> DNA
   <213> Artificial sequence
<220>
   <223> Description of artificial sequence: synthetic primer
<400> 5
   ggggacaagt ttgtacaaaa aagcaggctg ctctgtacag tgaccgg 47
<210> 6
   <211>
   <212> DNA
   <213> Artificial sequence
<220>
   <223> Description of artificial sequence: synthetic primer
<400> 6
   ggggaccact ttgtacaaga aagctgggtt ggtatggggc catccagag 49
<210> 7
   <211> 48
   <212> DNA
   <213> Artificial sequence
<220>
   <223> Description of artificial sequence: synthetic primer
<400> 7
   ggggacaact ttgtatagaa aagttgcaca acaacattca cggttggg 48
<210> 8
   <211> 48
   <212> DNA
   <213> Artificial sequence
<220>
   <223> Description of artificial sequence: synthetic primer
<400> 8
   ggggactgct tttttgtaca aacttgcttg gttgaccgaa ctcacctg 48
<210> 9
   <211> 49
   <212> DNA
   <213> Artificial sequence
<220>
   <223> Description of artificial sequence: synthetic primer
<900> 9
   ggggacagct ttcttgtaca aagtggtttg cgatttatgt ccgtggacg 49
<210> 10
   <211> 46
   <212> DNA
   <213> Artificial sequence
<220>
   <223> Description of artificial sequence: synthetic primer
<400> 10
   ggggacaact ttgtataata aagttgcata gtgatatgta cccgggc 46
<210> 11
   <211> 25
   <212> DNA
   <213> Artificial sequence
<220>
   <223> Description of artificial sequence: synthetic primer
<400> 11
   ccaggacgtc acgaacgaca tcaac 25
<210> 12
   <211> 18
   <212> DNA
   <213> Artificial sequence
<220>
   <223> Description of artificial sequence: synthetic primer
<400> 12
   cgcgccgggt actcgctc 18
<210> 13
   <211> 21
   <212> DNA
   <213> Artificial sequence
<220>
   <223> Description of artificial sequence: synthetic primer
<400> 13
   acaggtgact ctggatggcc c 21
<210> 14
   <211> 23
   <212> DNA
   <213> Artificial sequence
<220>
   <223> Description of artificial sequence: synthetic primer
<400> 14
   ttgacgagca cacctctacc agg 23
<210> 15
   <211> 435
   <212> DNA
   <213> Artificial sequence
<220> 435
   <223> Description of artificial sequence: synthetic primer
<400> 15
   cgggcccccc ctcgacggta tcgattggaa tgcatggagg agttctgtac gcgcaattcc    60
   gctctccgta aggatgcttc ggaggtgcac atggtctcat acatgtaggc ccgacgagga   120
   tcgagtcggt tccgaagtag gatcgtctcg attgttgggc atcattgcat ggacattcag   180
   agggcctact gatacctgga atccgcaccg tccggctacc tagcaataag attctgtgta   240
   tataaagggc taaggtgtcc gtccttgata aaaccaccac cctcaacaac ttacctcgac   300
   tatcagcatc ccgtcctatc taacaatcgt ccatcggtat ccaactccaa ctctattcgc   360
   agggtcctag aatcgtaagt acacgcttat atcttgttgc cagcgatagc tgacaatgaa   420
   tgaatatagg ccatg                                               435
<210> 16
   <211> 46
   <212> DNA
   <213> Artificial sequence
<220>
   <223> Description of artificial sequence: synthetic primer
<400> 16
   ggggacaact ttgtatagaa aagttgggcg gttcatctgc caacgc 46
<210> 17
   <211> 49
   <212> DNA
   <213> Artificial sequence
<220>
   <223> Description of artificial sequence: synthetic primer
<400> 17
   ggggactgct tttttgtaca aacttgcacc ctcttctttc agtcgtttg 49
<210> 18
   <211> 49
   <212> DNA
   <213> Artificial sequence
<220>
   <223> Description of artificial sequence: synthetic primer
<400> 18
   ggggacagct ttcttgtaca aagtggcacg acacgtgggg aatgatcag 49
<210> 19
   <211> 45
   <212> DNA
   <213> Artificial sequence
<220>
   <223> Description of artificial sequence: synthetic primer
<400> 19
   ggggacaact ttgtataata aagttgctct atcacacgtg gcgaac 45
<210> 20
   <211> 49
   <212> DNA
   <213> Artificial sequence
<220>
   <223> Description of artificial sequence: synthetic primer
<400> 20
   ggggacaact ttgtatagaa aagttgggcg ccgccgcaca aacccttcc 49
<210> 21
   <211> 50
   <212> DNA
   <213> Artificial sequence
<220>
   <223> Description of artificial sequence: synthetic primer
<400> 21
   ggggactgct tttttgtaca aacttgatca ttagagctga gctcaatcgc 50
<210> 22
   <211> 49
   <212> DNA
   <213> Artificial sequence
<220>
   <223> Description of artificial sequence: synthetic primer
<400> 22
   ggggacagct ttcttgtaca aagtgggttt gacagttaag aaagggaag 49
<210> 23
   <211> 48
   <212> DNA
   <213> Artificial sequence
<220>
   <223> Description of artificial sequence: synthetic primer
<400> 23
   ggggacaact ttgtataata aagttgcgga gaggtttccc actgcagac 48

## Description

### Field of the invention

The present invention relates to an improved method for efficient and targeted integration of nucleic acids into chromosomes of cells.

### Detailed description of the invention

Different cell types are used for different industrial purposes. For example mammalian cell lines are used for antibody production; fungal cells are preferred organisms for production of polypeptides and secondary metabolites; bacterial cells are preferred for small metabolite and antibiotic production; plant cells are preferred for taste and flavor compounds. Recombinant techniques are widely employed for optimization of the productivity of cells and/or processes. This can involve a multitude of options, including, but not limited to over expression of a gene of interest, deletion or inactivation of competing pathways, changing compartmentalization of enzymes, increasing protein or metabolite secretion, increasing organelle content and the like (see for example Khetan and Hu (1999) In: Manual of Industrial Microbiology Biotechnology, Eds. Demain and Davies, pg. 717-724). To be successful with these methods it is crucial that the recombinant construct is stably maintained in the production host. This can be either as part of an episomal vector or via integration in the genome. The latter situation is the preferred solution as this is the most stable situation. Even more preferred is the integration at the predetermined, correct genomic locus. Since in several species, especially most eukaryotic organisms, integration of DNA into the genome occurs with high frequency at random, the construction of industrial production cells by recombinant DNA technology often leads to the unwanted integration of the polynucleotide resulting in genome modifications at random. Moreover, this often results in multiple integrations and thus instable situations. This uncontrolled "at random multiple integration" of a polynucleotide is a potentially dangerous process, which can lead to unwanted modification of the genome of the host, resulting in decreased productivity.

It is therefore highly desirable to be able to construct an industrial production cell line by correct genome targeting of the polynucleotide sequence of interest with high efficiency. Furthermore, now that the sequences of complete genomes of an increasing amount of organisms are becoming available, the opportunity to construct genome-wide over expression and deletion libraries is opened. An important requirement for the efficient construction of such libraries is that the organism in question can be efficiently transformed, that the polynucleotide of interest is correctly targeted with a high frequency and that the required homology needed to direct targeted integration of a nucleic acid into the genome is relatively short.

There are several methods described to decrease the frequency of this unwanted, at random integration of polynucleotides in cells.

Eukaryotic cells have at least two separate pathways (one via homologous and one via non-homologous recombination) through which nucleic acids (in particular of course DNA) can be integrated into the host genome. The yeast *Saccharomyces cerevisiae* is an organism with a preference for homologous recombination (HR). The ratio of homologous to non-homologous recombination (HR/NHR) of this organism may vary from about 0.9 to 1. Contrary to *Saccharomyces cerevisiae,* higher eukaryotic cells (including fungal, plant and mammalian) cells have a preference for non-homologous recombination (NHR). Among these, the HR/NHR ratio ranges between 0.0001 and 0.5. In such organisms, the targeted integration frequency is rather low. Also, the length of homologous regions flanking a polynucleotide sequence to be integrated into the genome of such organisms has to be relatively long, for example at least 2,000 base pairs for disrupting a single gene. The necessity of such flanking regions represents a heavy burden when cloning the DNA construct comprising said polynucleotide and when transforming the organism with it. Moreover, neighboring genes which lie within those flanking regions can easily be disturbed during the recombination processes following transformation, thereby causing unwanted and unexpected side-effects.

Recently, several publications describe the inhibition of the very efficient Non-Homologous End-Joining (NHEJ) pathway, the pathway responsible for random integration of polynucleotides in cells, as a method for improving the HR/NHR ratio (see for example Ninomiya et al., 2004, Proc. Natl. Acad. Sci. USA 101:12248-12253; Krappmann et al., 2006, Eukaryot. Cell. 5:212-215). It is potentially a very powerful method, resulting in very significant improvements (even up to 60-fold) of gene targeting efficiency.

However, there are still some drawbacks to this method. Firstly, it does not work for all species. For example, mammalian cells deficient in *ku70,* one of the components of the NHEJ pathway, have been isolated (Pierce et al., Genes and Development, (2001), 15: 3237-3242). These mutants have a six-fold higher homology-directed repair frequency, but no increase in the efficiency of homology-directed targeted integration. Secondly, although it has a positive effect on the NHR/HR ratio in several fungal species (see for example Ninomiya *et al..,* 2004; Krappmann *et al..,* 2006) in most cases it is limited to 60-90% correct gene targeting. This is an acceptable improvement for working with one or several genes, but not for a High Throughput genome wide analysis and/or modification of gene function. In the individual cases were 100% correct transformants were obtained this involves long flanking regions, which also is not amenable for a High Throughput genome wide analysis and/or modification of gene function. Thirdly, to obtain such strains with improved HR/NHR ratios, one has to modify the recombination machinery of the host cell and this can lead to unwanted side effects (see for example Celli et al., Nat Cell Biol (2006), 8: 885-890).

The HR/NHR ratio can also be improved by over expressing components of the HR pathway. An example of this method is given by Shaked et al. (2005, Proc Natl. Acad. Sci. USA. 102:12265-12269). They show that by over expression of yeast RAD54 the HR frequency can be improved a 100-fold. Still, this results only 1-10% correct transformants, which makes this method not amenable for a High Throughput genome wide analysis and/or modification of gene function.

Another method is the so-called bipartite gene-targeting method (Nielsen *et al..,* 2006, 43: 54-64). This method is using two overlapping non-functional parts of a selection marker. Upon correct homologous recombination the selection marker becomes functional. They tested the method in the fungal species with the most efficient homologous recombination system, *Aspergillus nidulans,* with 24% correct gene targeting in WT cells. The method results in a 2.5-fold improvement over the standard method, but even in *Aspergillus nidulans* only 62% of the transformants obtained is correct. Also, rather long flanking regions are used to obtain correct targeting. This is an acceptable improvement for working with one or several genes, but not for a High Throughput genome wide analysis and/or modification of gene function.

Liu et al. (J. Bacteriol. 2001, 183: 1765-1772) describe another method, which uses a second selection marker to enrich for transformants with targeted gene disruption in *Acremonium chrysogenum.* The method results in a 10-fold improvement over the standard method, but still only 8% of the transformants obtained is correct.

Still another method is described by Kang and Khang (US 2005/0181509). This is a variation on the method of Liu *et al.* (2001). Here they apply a negative selection marker, *i.e*. the herpes simplex virus thymidine kinase (*HSVtk*) gene, as the second selection marker. If the selection procedure would work correctly, polynucleotides that integrate at random in the genome would kill the cells as the *HSVtk* gene would convert the 5-fluoro-2'-deoxyurine in the agar plates to a toxic compound. Again, this method increases the frequencies of correct targeting in cells, but it is limited to 50% of the cells. More importantly there is a very high percentage of false positives obtained (9-100%), which makes this method unsuitable for a High Throughput genome wide analysis and/or modification of gene function.

Kang and Khang (US 2005/0181509) also describe the testing of the diphtheria toxin A (*dtA*) gene. This gene has been applied in plants and mammalian cells as a second marker to increase the frequency of correct gene targeting to 1-2% (see for examples Terada et al., 2004, Plant Cell Rep. 22:653-659; Yagi et al., 1993, Anal. Biochem. 214:77-86). However, they failed to get this marker functional in fungal species.

Surprisingly, we found that the diphtheria toxin A (*dtA*) gene does work in filamentous fungal cells and can be used efficiently in fungal species as a lethal marker to enrich for cells wherein a correct gene targeting event has occurred.

The present invention discloses a method to construct fungal cells having a target sequence in a chromosomal DNA sequence replaced by a desired replacement sequence in any genetic background, including wild type cells, comprising:
providing a DNA molecule comprising a first DNA fragment comprising a desired replacement sequence flanked at its 5' and 3' sides by DNA sequences substantially homologous to sequences of the chromosomal DNA flanking the target sequence and a second DNA fragment comprising an expression cassette comprising a gene encoding diphtheria toxin A and regulatory sequences functional in the fungal cell operably linked thereto;
transforming the fungal cells with the resulting DNA molecule;
growing the cells to obtain transformed progeny cells having the DNA molecule inserted into the chromosome, wherein cells in which the DNA molecule is inserted in the chromosome via a non-homologous recombination event are selectively killed by expression of diphtheria toxin A; and
obtaining cells wherein the target sequence in the chromosomal DNA sequence is replaced by the desired replacement sequence_{.}
wherein the substantially homologous DNA sequences flanking the replacement sequence have a degree of identity to a chromosomal DNA sequence flanking the target sequence of at least 80% over a region of not more than 3 kb, and wherein the fungal cells are of the genus *Aspergillus, Penicillium, Acremonium, Trichoderma, Chrysosporium, Mortierella, Kluyveromyces, Sacchararomyces* or *Pichia;* preferably of the species *Aspergillus niger, Aspergillus nidulans, Aspergillus oryzae, Aspergillus terreus, Penicillium chrysogenum, Penicillium citrinum, Acremonium chrysogenum, Trichoderma reesei, Mortierella alpina, Chrysosporium lucknowense, Kluyveromyces lactis, Saccharomyces cerevisiae, Pichia pastoris* or *Pichia ciferrii.*

The first DNA fragment comprises a desired replacement sequence flanked at its 5' and 3' sides by DNA sequences substantially homologous to sequences in the chromosomal DNA flanking the target sequence.

With the term "substantially homologous" as used in this invention is meant that a DNA sequence flanking the replacement sequence has a degree of identity to a chromosomal DNA sequence flanking the target sequence of at least 80%, preferably at least 90%, over a region of not more than 3 kb, preferably not more than 2 kb, more preferably not more than 1 kb, even more preferably not more than 0.5 kb, even more preferably not more than 0.2 kb. even more preferably not more than 0.1 kb, even more preferably not more than 0.05 kb, most preferably not more than 0.03 kb. The degree of required identity may thereby depend on the length of the substantially homologous sequence. The shorter the homologous sequence, the higher the percentage homology may be.

It will be obvious to the skilled person that, in order to achieve homologous recombination via a double cross-over event, these flanking sequences need to be present at both sides of the replacement sequence and need to be substantially homologous to sequences at both sides of the target sequence in the chromosome.

The nature of the replacement sequence may vary depending on the intended use. The replacement sequence may for instance confer a selectable phenotype to the fungal cell. In that case, the replacement sequence comprises a selection marker. Preferably, the selection marker is a positive selection marker. A preferred positive selection marker is the *amdS* gene. A selection marker as replacement sequence preferably is used when the target sequence needs to be inactivated.

The replacement sequence may also be a modified version of the target sequence, for instance to provide for altered regulation of a sequence of interest or expression of a modified gene product with altered properties as compared to the original gene product.

The replacement sequence may also constitute additional copies of a sequence of interest being present in the genome of the fungal cell, to obtain amplification of that sequence of interest.

The replacement sequence may be a sequence homologous or heterologous to the fungal cell of interest. It may be obtainable from any suitable source or may be prepared by custom synthesis.

The target sequence may be any sequence of interest. For instance, the target sequence may be a sequence of which the function is to be investigated by inactivating or modifying the sequence. The target sequence may also be a sequence of which inactivation, modification or over expression is desirable to confer a fungal strain with a desired phenotype.

The second DNA fragment comprises an expression cassette providing for expression of the diphtheria toxin A. However, only a non-homologous recombination event will lead to actual integration of the diphtheria toxin A cassette. This implicates that expression of the toxin will only occur upon integration of the DNA molecule comprising the first and second DNA fragment into the chromosome of the fungal cell via non-homologous recombination. Integration of the expression cassette thus will only occur when the DNA molecule is integrated at a site that is not homologous to the target sequence.

The expression cassette providing for expression of the diphtheria toxin A comprises regulatory sequences operably linked to the diphtheria toxin A-encoding *dtA* gene. The term "operably linked" refers to a juxtaposition wherein the components described are in a relationship permitting them to function in their intended manner. A regulatory sequence such as a promoter, an enhancer or another expression regulatory signal "operably linked" to a coding sequence is positioned in such a way that expression of a polypeptide from its coding sequence is achieved under conditions compatible with the regulatory sequences.

The regulatory sequences of the *dtA* expression cassette preferably are heterologous to the chromosome of the fungal cell of interest, *i.e*. the regulatory sequences are from a different fungal species than the fungal cell of interest to be transformed. The use of a homologous regulatory sequence in this context may result in a targeted integration event at a chromosomal site corresponding to the homologous regulatory sequence. Such an integration event is undesirable because it decreases the percentage correct targeting to the site comprising the targeting sequence.

Regulatory sequences used may drive constitutive expression; this will enable to expression of the negative selection directly after transfection. Alternatively, regulatory sequences may be used that drive regulable or inducible expression of the *dtA* gene; this allows for a two step procedure. Firstly, the transfection and subsequent isolation of transformants is performed under conditions that expression of the *dtA* gene does not occur. Secondly, the isolated transformants are transferred to conditions which induce the expression of the *dtA* gene, thereby selectively killing all the isolates that underwent random integration events.

The DNA molecule may comprise the first and second DNA fragment in any order and preferably is a linear molecule. If the replacement sequence does not comprise a selection marker, such a marker may be provided on a separate DNA molecule.

A fungal cell of interest is transformed with the DNA molecule comprising the first and second DNA fragment, and, optionally, a DNA molecule comprising a selection marker, using techniques commonly known in the art. Briefly, fungal cells are transformed by contacting the fungal cells with a suitable amount of the DNA molecule(s), preferably in linear form, and selecting colonies of transformed cells by culturing the cells on a selective medium enabling growth of transformed cells only.

Upon transformation, the DNA molecule comprising the first and second DNA fragment integrates in the chromosome of the fungal host cell by a homologous or a non-homologous integration event. A homologous integration event occurs at the target sequence in the host chromosome by a double cross-over event at the homologous sequences flanking the replacement and targeting sequence. Such an event ensures that the second DNA fragment comprising the *dtA* expression cassette is not integrated into the chromosome. Alternatively, a single cross-over event at one of the homologous flanking sequences can occur, resulting in the integration of the full DNA fragment (including first and second marker). However, due to the co-integration of the *dtA* expression cassette. A non-homologous integration event results in integration of the complete DNA molecule comprising first and second DNA fragments. Cells wherein either a homologous single-cross over or a non-homologous integration event has occurred are selectively killed when the *dtA* gene is expressed upon integration. This expression of the *dtA* gene may occur simultaneously with selection of the transformants or may occur in a later stage after transformants have been selected. In the latter case, expression of the *dtA* gene may be not be constitutive but induced by a suitable inducer.

The fungal cell may be any fungal cell of interest. Preferably, the fungal cell is of the genus *Aspergillus, Penicillium, Acremonium, Trichoderma, Chrysosporium, Mortierella, Kluyveromyces, Saccharomyces* or *Pichia;* more preferably of the species *Aspergillus niger, Aspergillus nidulans, Aspergillus oryzae, Aspergillus terreus, Penicillium chrysogenum, Penicillium citrinum, Acremonium chrysogenum, Trichoderma reesei, Mortierella alpina, Chrysosporium lucknowense, Kluyveromyces lactis, Saccharomyces cerevisiae, Pichia pastoris* or *Pichia ciferrii.*

The method of the invention advantageously allows the provision of transformed fungal cells that are enriched in cells wherein the correct targeted integration event has occurred. In particular, at least 50% of the transformed colonies has the replacement sequence targeted to the target sequence in the chromosome, as compared to 1-2% in a transformation with a targeting construct without a second fragment comprising the *dtA* gene.

### Description of the Figures

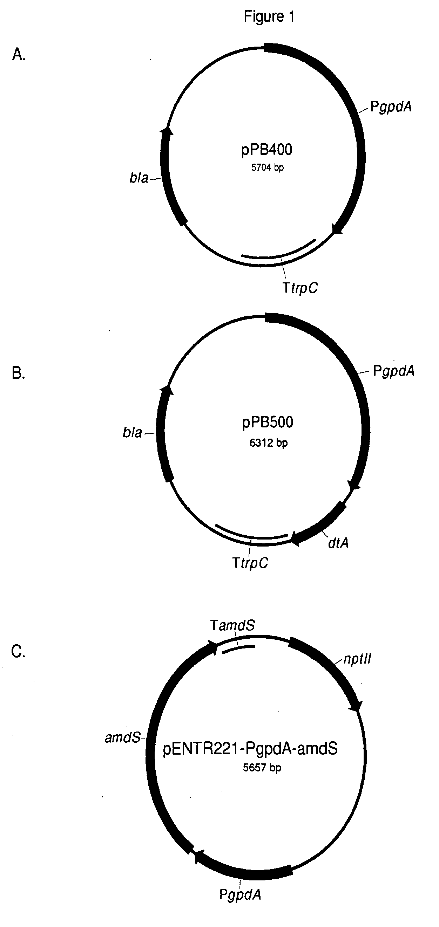

Figure 1 shows the vectors pPB400 (1A), pB500 (1B) and pENTR221-PgpdA-An*amdS*.

Legend: P*gpdA* = *Aspergillus nidulans gpdA* promoter; *dtA* = Corynebacterium diphtheria toxin-A gene; T*trpC* = *Aspergillus nidulans trpC* terminator; *bla* = β-lactamase gene, *amdS = Aspergillus nidulans amdS* gene; *nptll* = kanamycine resistance gene; T*amdS* = *Aspergillus nidulans amdS* terminator.

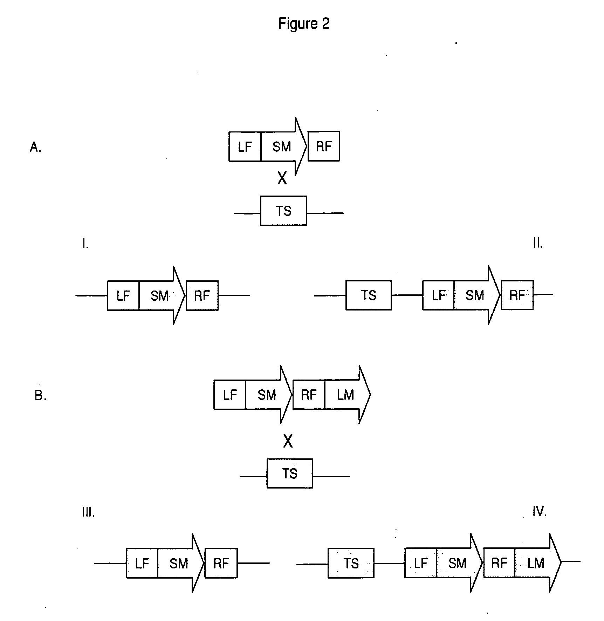

Figure 2 shows the recombination options during chromosomal integration. 2A: transfection of a `classical' gene targeting construct with two flanking regions to direct the selection marker (SM) to the target sequence (TS). Either the gene targeting is successful after a double homologous cross over exchanging the TS for the SM (option I.) or the integration occurs ectopically in which situation both the TS and the SM reside in the genomic DNA (option II.). A third option, the single cross over process, is not depicted but basically results in the same as the latter situation. In filamentous fungi the ratio between option I. and option II. is 1:10 to 1:10,000 (depending on the species used). 2B: transfection of a *dtA*-facilitated gene targeting construct with two flanking regions to direct the SM to the TS locus and the *dtA* gene (*i.e*. lethal selection marker, LM) to select against ectopic and single-cross over integration events. Again, either the gene targeting is successful after a double homologous cross over exchanging the TS for the SM and the *dtA* gene is lost (option III.) or the integration occurs ectopically in which situation the TS, the SM and the LM integrate in the genomic DNA (option IV.). In the latter case the *dtA* gene becomes transcribed and the expression of the toxin-A protein causes cell death, thereby automatically deselecting the unwanted integration events. In fungi the ratio between option III. and option IV. is shifted towards 1:0 to 1:20.

Legend: LF = homologous flanking region to the left of the target sequence; SM = selection marker gene cassette; RF = homologous flanking region to the right of the target sequence; TS = target sequence; LM = lethal selection marker (*i.e*. the *dtA* gene).

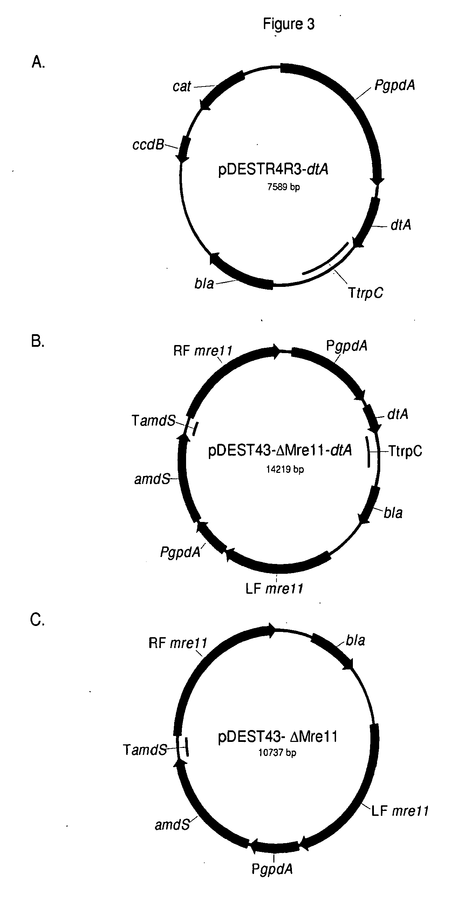

Figure 3 shows the vectors pDESTR4R3-*dtA* (3A), pDEST43-Δmre11-*dtA* (3B) and pDEST43-Δmre11 (3C).

Legend: P*gpdA* = *Aspergillus nidulans gpdA* promoter; *dtA* = *Corynebacterium diphtheria* toxin-A gene; T*trpC* = *Aspergillus nidulans trpC* terminator; *bla* = β-lactamase gene; *mre11* = *Penicillium chrysogenum mre11* locus; *amdS* = *Aspergillus nidulans* acetamidase gene; T*amdS* = *Aspergillus nidulans amdS* terminator; LF= left flank or 5' targeting sequence; RF = right flank or 3' targeting sequence; *cat* = chloramphenicol resistance gene; *ccdB* = DNA gyrase gene.

Figure 4 shows schematically the annealing position of the oligonucleotides used in the colony PCR for verification of correct gene replacement of the *Penicillium chrysogenum mre11* gene. 4A. In the correct situation both combinations of oligonucleotides (5' flank fwd plus 5' flank rev and 3' flank fwd and 3' flank rev) will give a PCR amplified fragment. 4B. In the wild type situation and/or non-targeted integration events there will be no amplification for both oligonucleotide combinations.

Figure 5 shows the vector pPB600.

Legend: P*toxA* = *Pyrenophora tritici-repentis toxA* promoter; *dtA* = *Corynebacterium diphtheria* toxin-A gene; T*trpC* = *Aspergillus nidulans trpC terminator, bla* = β-lactamase gene.

### Examples

### General materials and methods

Standard procedures were carried out as described elsewhere (Sambrook et al., 1989, Molecular cloning: a laboratory manual, 2nd Ed., Cold Spring Harbor Laboratory Press, Cold Spring Harbor, NY). DNA for plasmid construction was amplified using the proofreading polymerases, following the manufacturer's protocol; while verification of constructed strains and plasmids was achieved using Taq polymerase. Restriction enzymes were from Invitrogen or New England Biolabs. For routine cloning, *Escherichia coli* strains Top10 and DH10B (Invitrogen) were used. The Gateway system of Invitrogen was applied according to the manufacturer's manuals. Verification of the constructed plasmids was carried out by restriction analysis and subsequent sequencing.

### Example 1

### Use of the negative selection marker diphteria toxin A-chain in Penicillium chrysogenum during co-transformation

In order to get the gene encoding the *Corynebacterium diphteriae* toxin A-chain functionally expressed in filamentous fungi it was cloned downstream of a commonly used fungal promoter: the *Aspergillus nidulans gpdA* promoter. To this end the pAN7-1 (Punt et al., 1987, Gene 56: 117-124) was modified as follows. First, the *hph* gene, encoding the hygromycin B resistance marker, had to be deleted. A PCR fragment which should replace the *hph* gene containing the 3' part of the *Aspergillus nidulans gpdA* promoter (*i.e.* P*gpdA*)*,* followed by two newly introduced *Nco*I and *Not*I sites at the border of P*gpdA* and the *trpC terminator* (T*trpC*)*,* and the *Bam*HI site at the border between the *hph* gene and P*trpC,* was produced using the oligonucleotides of SEQ ID NO 1 and SEQ ID NO 2. The obtained PCR fragment was digested with *Sal*I and *Bam*HI and the resulting 343 bp fragment was used to replace the 1395 bp Sa*l*I and *Bam*HI fragment from pAN7-1, thus replacing the *hph* gene and creating pPB400 (Fig. 1A). Next, the *dtA* gene was PCR amplified from the pTHH-plasmids (Breitman et al., 1990, Mol Cell Biol 10: 474-479) using the oligonucleotides of SEQ ID NO 3 and SEQ ID NO 4 and cloned into pGEM-T Easy (Promega). From the resulting pGEM-T-dtA plasmid, the *Nco*I*-Not*I region encompassing the *dtA* gene was isolated and cloned into pPB400, also digested with *Nco*I and *Not*I*.* The resulting plasmid pPB500 now contains the *dtA* gene under the control of the *gpdA* promoter, followed by the *trpC* terminator (Fig. 1 B). *Penicillium chrysogenum* protoplasts were produced according to standard protocols (see for examples Cantoral et al., 1987, Biotechnology 5: 494-497; Swinkels et al., 1997, WO97/06261) however Glucanex^{™} (Sigma) was applied as lysing enzyme. To test the efficiency of the DtA toxin to operate as a functional negative selection marker DNA was transfected to these protoplasts in different combinations (Table 1). As a positive control pENTR221-PgpdA-*amdS* was used; this contains a positive selection marker amdS driven by the heterologous promoter from the *Aspergillus nidulans* gpdA gene. This plasmid was constructed as follows. The P*gpdA-AnamdS-*T*trpC* gene cassette was PCR amplified from the plasmid pAN7-1 by using oligonucleotides SEQ ID NO 5 and SEQ ID NO 6. These include the sequences for the so-called Gateway Entry reaction (*i.e*. attB1 and attB2, see Gateway manuals on www.Invitrogen.com). The fragment was recombined using Invitrogen's clonase enzymes into the donor vector pDONR221, resulting in pENTR221-PgpdA-*amdS* (See Fig. 1C).

After transfection with the various DNA combinations the protoplasts were plated out on selective regeneration agar plates with acetamide as the sole nitrogen source (for an exact description of the media, see Swinkels et *al.,* 1997). Co-transformation of *P. chrysogenum* using two plasmids of which only one contains a selectable marker, can result in efficiencies of up to 90% (Kolar et al., 1988, Gene 62: 127-134), meaning that in 90% of the transformants, the second, non-selectable plasmid has also been taken up by the protoplasts. This trait was used in co-transformation experiments in which in addition to 2.5 µg of pENTR221-gpdA::*amdS*, also 2.5 µg of plasmid pPB500, containing the *dtA* gene of *C*. *diphteriae* driven by the *Aspergillus nidulans gpdA* promoter (Fig. 1C), was added to the protoplasts. Once plasmid pPB500 is taken up and the *dtA* gene is transcribed, the cell is expected to die. Indeed, after addition of the *dtA* plasmid, the number of transformants was almost 7-fold lower than when only the *amdS* plasmid was added to the protoplasts (Table 1).

The negative effect when a plasmid containing the *dtA* gene is co-transformed alongside pENTR221-PgpdA-*amdS* could theoretically also be due to a competitive effect during uptake of the DNA by the protoplasts. Therefore, an almost identical plasmid to pPB500, just lacking the *dtA* gene (pPB400, Fig. 1 B), was also used for co-transformation of *P. chrysogenum.* The addition of pPB400 as second plasmid in co-transformation of *P. chrysogenum* had a negative effect on the transformation frequency, but this effect was less profound when compared to the negative effect of pPB500: 3-fold vs. 7-fold (Table 1). Together, these data strongly suggest that *dtA* is lethal to *P*. *chrysogenum,* thus enabling the use of *dtA* as negative selection marker in *P. chrysogenum* transformations.

**Table 1. Number of transformants after (co)-transformation of P. chrysogenum protoplasts. In all cases pENTR221-PgpdA-amdS was used to select on acetamide.**

| | co-transformed plasmid | | |
|---|---|---|---|
| | None | pPB500 | pPB400 |
| Number of transformants | 1700 | 260 | 535 |

### Example 2

### Gene-Targeting in Penicillium chrysogenum using dtA as a negative selection marker

The results described above suggest that the *dtA* gene could act as negative selection marker; therefore it was tested if *dtA* expressed from the *Aspergillus nidulans gpdA* promoter could be used as a true negative selection marker in fungi, like a killer gene. Although, recent reports claim that this is not possible (US 2005/0181509), we believed it should be possible to apply *dtA* as a negative selection marker to deselect for unwanted DNA integration events (see Fig. 2). First, the P*gpdA-dfA-TtrpC* construct from plasmid pPB500 was obtained as a blunt-ended fragment after *Bgl*II and *Xba*I digestion and subsequent Klenow fill-in. The fragment was cloned into the blunted Ndel (via Klenow fill-in treatment) site of destination vector pDESTR4-R3, which is part of the Invitrogen Multisite Gateway® system. Next, this pDESTR4-R3-*dtA* destination vector (Fig. 3A) was combined with entry clones containing 2.5 kb up- and downstream flanks of the *P*. *chrysogenum mre11* gene and the *gpdA::amdS* selectable marker in an LR recombination reaction according to the manufacturers instructions, resulting in gene targeting construct pDEST43-Δmre11-*dtA* (Fig. 3B). As a control the vector pDEST43-Δmre11 was constructed by recombining the 2.5 kb up- and downstream flanks of the *P. chrysogenum mre11* gene and the *gpdA::amdS* selectable marker in an LR recombination reaction into the destination vector pDESTR4-R3, thereby obtaining an almost identical plasmid, but lacking the *dtA* gene (Fig. 3C). The up- and downstream flanks of the *P. chrysogenum mre11* gene were obtained by PCR amplification using the oligonucleotides SEQ ID NO 7 plus SEQ ID NO 8 and SEQ ID NO 9 plus SEQ ID NO 10, respectively. The obtained 2.5 kb fragments were cloned via recombination into the pDONRP4-P1 R and pDONRP2R-P3, respectively.
Using a killer gene in such a way is based on survival upon DNA integration. When true gene targeting via double homologous cross-over occurs, only the *gpdA::amdS* fragment will be inserted into the genome of the recipient. The *dtA* gene will subsequently be degraded, enabling the cell to survive. However, when single cross-over at either the 5'-or 3'-flank does occur, the *dtA* gene will also be integrated into the genome, thus killing that particular cell. Likewise, when the donor DNA integrates ectopically, the *dtA* gene will also be inserted, again killing the recipient. By comparing the two almost identical constructs, one containing *dtA* as a negative selection marker (pDEST43-Δmre11-*dtA*, Fig. 3B), the other lacking the *dtA* gene (pDEST43-Δmre11, Fig. 3C), the usefulness of *dtA* as a direct negative selection marker in fungi was assessed. *P*. *chrysogenum* protoplasts were prepared and transfected as described in example 1 with 5 µg of each plasmid and selected for growth on acetamide regeneration plates. A strong reduction in transformants was observed when *dtA* was used in comparison to when no *dtA* was used: ~60 and ~1900 primary transformants were obtained respectively (see Table 2).

**Table 2. Number of transformants after transfection of P. chrysogenum protoplasts with or without dtA (i.e. using pDEST43-Δmre11-dtA and pDEST43-Δmre11, respectively).**

| | 1^{st} experiment | | 2^{nd} experiment | |
|---|---|---|---|---|
| | *+dtA* | *-dtA* | *+dtA* | *-dtA* |
| Number of transformants | 69 | ±2400 | 51 | ±1400 |

This huge decrease in surviving transformants when *dtA* was included as negative selectable marker is most likely caused by the killing of regenerated protoplasts in which the DNA has integrated ectopically or via a single homologous cross-over. Hence the *dtA* can be efficiently used as a dominant negative selectable marker in fungi.

### Example 3

### Genomic analysis of Penicillium chrysogenum transformants obtained by using dtA as a negative selection marker

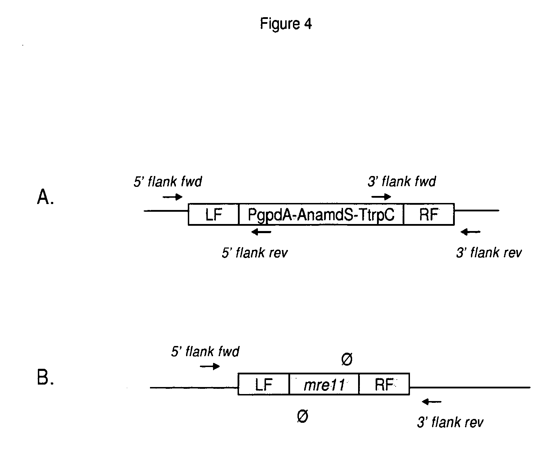

To determine if the surviving transformants obtained as described in example 2 are correct gene replacements (so exact gene targeting has taken place) a set of colony PCR's was performed. Stable transformants were obtained after spotting the primary transformants of example 2 on fresh acetamide plates without saccharose to induce sporulation. These candidate isolates were grown on agar plates for 4-6 days and used to make cell suspensions in water. DNA was liberated by boiling the suspension for 10 minutes. A small amount of the cleared supernatant was used as a template DNA for PCR amplification. In order to determine correct gene replacement oligonucleotides annealing outside the flanking regions used in the transfection were combined with oligonucleotides annealing to the *amdS* cassette (see Fig. 4). In practice, this means that a 5' flank PCR was performed using the oligonucleotides SEQ ID NO 11 plus SEQ ID NO 12 and a 3' flank PCR was performed using the oligonucleotides SEQ ID NO 13 plus SEQ ID NO 14. Indeed, PCR analysis of both analyzed surviving transformants after the use of *dtA*, showed correct gene targeting at the *mre11* locus for both flanks, while all *amdS*-positive transformants obtained from the transfection without *dtA* gave no PCR results, clearly demonstrating the strong enhancing effect of the *dtA* killer gene on gene targeting efficiencies in fungi (see Table 3).

**Table 3. Colony PCR results after transfection of P. chrysogenum protoplasts with or without dtA.**

| Transfection origin | Colony number | 5' flank PCR | 3' flank PCR |
|---|---|---|---|
| | | SEQ ID NO 13+14 | SEQ ID NO 15+16 |
| *+dtA* | 5 | + | + |
| *+dtA* | 16 | + | + |
| *-dtA* | 1 | - | - |
| wild type cells | 1 | - | - |

### Example 4

### Use of the dtA gene in Penicillium chrysogenum during co-transformation

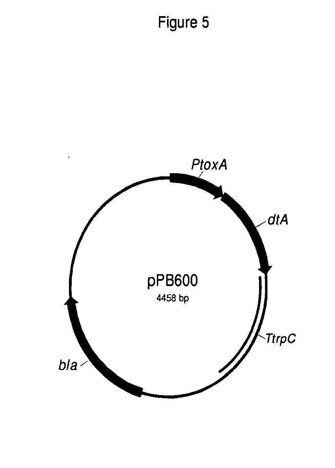

In order to determine if the effectiveness of *dtA* was merely due to the strong *Aspergillus nidulans gpdA* promoter a second independent promoter was used: the *Pyrenophora tritici-repentis toxA* promoter. First, the *toxA* promoter was obtained as a blunt-ended fragment (Ciufetti et al., 1997, Plant Cell 9:135-144), see SEQ ID NO 15. The *gpdA* promoter of pBP500 was removed as an *Eco*RI-*Nco*I fragment and the linear vector backbone was blunted. This backbone was used to ligate the blunt-ended P*toxA* fragment, yielding pBP600 (see Fig. 5). In this experiment each transfection was performed with 2.5 µg of pDEST43-Δmre11, which would enable acetamide selection, and either no DNA, 2.5 µg of pBP400 (=control), 2.5 µg of pBP500 (=P*gpdA-dtA*) or 2.5 µg of pBP600 (=P*toxA-dtA*). Transformants were selected for growth on acetamide regeneration plates.

**Table 5. Number of transformants after transfection of P. chrysogenum protoplasts with pDEST43-Δmre11 and various plasmids (see text for further details). n.t. = not tested.**

| Experiment | Δ*mre11* | Δ*mre11* + pBP400 *(control)* | Δ*mre11* + pBP500 *(PgpdA-dtA)* | Δ*mre11* + pBP600 *(PtoxA-dtA)* |
|---|---|---|---|---|
| #1 | >200 | 135 | 75 | n.t. |
| #2 | > 200 | 104 | 24 | 51 |

The results clearly show a similar effect when using the *toxA* promoter of *Pyrenophora tritici-repentis* instead of the *Aspergillus nidulans gpdA* promoter, thereby demonstrating that the effectiveness of *dtA* in fungi is not depending on a single strong promoter.

### Example 5

### Gene targeting to the rad50 and dln4 loci in Penicillium chrysogenum using dtA as a negative selection marker

In order to determine if the results described above were locus dependent, two independent loci residing elsewhere in the genome were targeted for gene replacement using the same approach. First, the up- and downstream flanks of the *P. chrysogenum rad50* gene were obtained by PCR amplification using the oligonucleotides SEQ ID NO 16 plus SEQ ID NO 17 and SEQ ID NO 18 plus SEQ ID NO 19, respectively. The obtained 2.5 kb fragments were cloned via recombination into the pDONRP4-P1 R and pDONRP2R-P3, respectively. Secondly, the up- and downstream flanks of the *P. chrysogenum dln4* gene were obtained by PCR amplification using the oligonucleotides SEQ ID NO 20 plus SEQ ID NO 21 and SEQ ID NO 22 plus SEQ ID NO 23, respectively. The obtained 2.5 kb fragments were cloned via recombination into the pDONRP4-P1R and pDONRP2R-P3, respectively.
The thus obtained flanking regions were recombined with the *amdS* gene cassette from pDONR221-gpdA::*amdS* into the two version of the destination vector (with and without *dtA*, see Fig. 3) to form the pDEST43-Δrad50-*dtA*, pDEST43-Δrad50, pDEST43-Δdln4-*dtA* and pDEST43-Δdln4. Again 5 µg of each of these plasmids were transfected to *Penicillium chrysogenum* protoplasts and transformants were selected for growth on acetamide regeneration plates.

**Table 6. Number of transformants after transfection of P. chrysogenum protoplasts with various gene targeting constructs, plus or minus dtA.**

| Gene locus | | 1^{st} experiment | | 2^{nd} experiment | |
|---|---|---|---|---|---|
| | | +*dtA* | -*dtA* | +*dtA* | -*dtA* |
| *rad50* | Number of transformants | 55 | ±1800 | 13 | ±1300 |
| *dln4* | Number of transformants | 36 | ±2100 | 43 | ±2100 |

The results clearly show an effect of the presence of the *dtA* gene. The number of surviving cells (*i.e*. transformants which underwent a putative correct gene targeting event) do well fit with the known percentage of transformants with correct gene targeting in a `classical' experimental set-up, namely 1-3%. The use of *dtA* deselects the ectopic and single cross-over integrants and thereby increases the percentage of transformants with correct gene targeting well over 25%.

## Claims

1. A method to construct fungal cells having a target sequence in a chromosomal DNA sequence replaced by a desired replacement sequence, comprising:
providing a DNA molecule comprising a first DNA fragment comprising a desired replacement sequence flanked at its 5' and 3' sides by DNA sequences substantially homologous to sequences of the chromosomal DNA flanking the target sequence and a second DNA fragment comprising an expression cassette comprising a gene encoding diphtheria toxin A and regulatory sequences functional in the fungal cell operably linked thereto;
transforming the fungal cells with the resulting DNA molecule;
growing the cells to obtain transformed progeny cells having the DNA molecule inserted into the chromosome, wherein cells in which the DNA molecule is inserted in the chromosome via a non-homologous recombination event are selectively killed by expression of diphtheria toxin A; and
obtaining cells wherein the target sequence in the chromosomal DNA sequence is replaced by the desired replacement sequence, wherein the substantially homologous DNA sequences flanking the replacement sequence have a degree of identity to a chromosomal DNA sequence flanking the target sequence of at least 80% over a region of not more than 3 kb and wherein the fungal cells are of the genus *Aspergillus, Penicillium, Acremonium, Trichoderma, Chrysosporium, Mortierella, Kluyveromyces, Sacchararomyces* or *Pichia;* preferably of the species *Aspergillus niger, Aspergillus nidulans, Aspergillus oryzae, Aspergillus terreus, Penicillium chrysogenum, Penicillium citrinum, Acremonium chrysogenum, Trichoderma reesei, Mortierella alpina, Chrysosporium lucknowense, Kluyveromyces lactis, Saccharomyces cerevisiae, Pichia pastoris* or *Pichia ciferrii*.

2. The method according to claim 1, wherein the replacement sequence comprises a selection marker, a modified version of the target sequence and/or additional copies of a sequence of interest being present in the genome of the fungal cell.

3. The method according to any one of claims 1-2, wherein the regulatory sequences of the diphtheria toxin A expression cassette are heterologous to the fungal cell.

4. The method according to any one of claims 1-3, wherein the regulatory sequences of the diphtheria toxin A expression cassette comprise a constitutive or an inducible promoter.

## Patentansprüche

1. Verfahren für die Konstruktion von Pilzzellen, bei denen eine Zielsequenz in einer chromosomalen DNA-Sequenz durch eine gewünschte Ersatzsequenz ersetzt ist, umfassend die Schritte:
Bereitstellen eines DNA-Moleküls umfassend ein erstes DNA-Fragment umfassend eine gewünschte Ersatzsequenz, die 5'-seitig und 3'-seitig von DNA-Sequenzen flankiert wird, die zu Sequenzen der chromosomalen DNA, die die Zielsequenz flankiert, im Wesentlichen homolog sind, und ein zweites DNA-Fragment umfassend eine Expressionskassette umfassend ein Gen, das für das Diphtherietoxin A kodiert, und damit operativ verbundene Regulationssequenzen, die in der Pilzzelle funktionell sind;
Transformieren der Pilzzellen mit dem erhaltenen DNA-Molekül;
Züchten der Zellen zwecks Erhalt von transformierten Nachkommenschaftszellen, bei denen das DNA-Molekül in das Chromosom insertiert vorliegt, wobei Zellen, in denen das DNA-Molekül in das Chromosom über ein nichthomologes Rekombinations-Event insertiert ist, durch Expression des Diphtherietoxins A selektiv abgetötet werden; und
Gewinnen von Zellen, bei denen die Zielsequenz in der chromosomalen DNA-Sequenz durch die gewünschte Ersatzsequenz ersetzt ist, wobei die im Wesentlichen homologen DNA-Sequenzen, die die Ersatzsequenz flankieren, mindestens 80% Identität über eine Region von maximal 3 kB zu einer chromosomalen DNA-Sequenz, die die Zielsequenz flankiert, aufweisen, und wobei die Pilzzellen zu der Gattung *Aspergillus,*
*Penicillium, Acremonium, Trichoderma, Chrysosporium, Mortierella, Kluyveromyces, Sacchararomyces* oder *Pichia,* vorzugsweise der Art *Aspergillus niger, Aspergillus nidulans, Aspergillus oryzae, Aspergillus terreus, Penicillium chrysogenum, Penicillium citrinum, Acremonium chrysogenum, Trichoderma reesei, Mortierella alpina, Chrysosporium lucknowense, Kluyveromyces lactis, Saccharomyces cerevisiae, Pichia pastoris* oder *Pichia ciferrii* gehören.

2. Verfahren nach Anspruch 1, wobei die Ersatzsequenz einen Selektionsmarker, eine modifizierte Version der Zielsequenz und/oder zusätzliche Kopien einer interessierenden Sequenz, die im Genom der Pilzzelle vorliegt, umfasst.

3. Verfahren nach einem der Ansprüche 1-2, wobei die Regulationssequenzen der Diphtherietoxin-A-Expressionskassette zu der Pilzzelle heterolog sind.

4. Verfahren nach einem der Ansprüche 1-3, wobei die Regulationssequenzen der Diphtherietoxin-A-Expressionskassette einen konstitutiven oder einen induzierbaren Promoter umfassen.

## Revendications

1. Méthode de construction de cellules fongiques ayant une séquence cible dans une séquence d'ADN chromosomique remplacée par une séquence de remplacement désirée comprenant:
l'obtention d'une molécule d'ADN comprenant un premier fragment d'ADN comprenant une séquence de remplacement désirée flanquée à ses côtés 5' et 3' par des séquences d'ADN sensiblement homologues à des séquences de l'ADN chromosomique flanquant la séquence cible et un deuxième fragment d'ADN comprenant une cassette d'expression contenant un gène codant pour la toxine diphtérique A et des séquences de régulation fonctionnelles dans la cellule fongique qui y sont liées de façon opérationnelle ;
la transformation des cellules fongiques avec la molécule d'ADN résultante ;
la mise en culture des cellules pour obtenir des cellules de la progéniture transformées ayant la molécule d'ADN insérée dans le chromosome, les cellules dans lesquelles la molécule d'ADN est insérée dans le chromosome par l'intermédiaire d'un évènement de recombinaison non homologue étant sélectivement tuées par l'expression de la toxine diphtérique A ; et
l'obtention de cellules dans lesquelles la séquence cible dans la séquence d'ADN chromosomique est remplacée par la séquence de remplacement désirée, les séquences d'ADN sensiblement homologues flanquant la séquence de remplacement ayant un degré d'identité avec la séquence d'ADN chromosomique flanquant la séquence cible d'au moins 80 % sur une région de pas plus de 3 kb, et
les cellules fongiques étant du genre *Aspergillus, Penicillium, Acremonium, Trichoderma, Chrysosporium, Mortierella, Kluyveromyces, Sacchararomyces* ou *Pichia ;* de préférence des espèces *Aspergillus niger, Aspergillus nidulans, Aspergillus oryzae, Aspergillus terreus, Penicillium chrysogenum, Penicillium citrinum, Acremonium chrysogenum, Trichoderma reesei, Mortierella alpina, Chrysosporium lucknowense, Kluyveromyces lactis, Saccharomyces cerevisiae, Pichia pastoris* ou *Pichia ciferrii.*

2. Méthode selon la revendication 1 **caractérisée en ce que** la séquence de remplacement comprend un marqueur de sélection, une version modifiée de la séquence cible et/ou des copies supplémentaires d'une séquence d'intérêt étant présentes dans le génome de la cellule fongique.

3. Méthode selon l'une ou l'autre des revendications 1 et 2 **caractérisée en ce que** les séquences de régulation de la cassette d'expression de la toxine diphtérique A sont hétérologues vis-à-vis de la cellule fongique.

4. Méthode selon l'une quelconque des revendications 1 à 3 **caractérisée en ce que** les séquences de régulation de la cassette d'expression de la toxine diphtérique A comprennent un promoteur constitutif ou inductible.
